# EUROPÄISCHE PATENTSCHRIFT

(11) **EP 0 414 263 B1**
(45) Veröffentlichungstag und Bekanntmachung des Hinweises auf die Patenterteilung: **26.10.1994**
(21) Anmeldenummer: 90116265.1
(22) Anmeldetag: 24.08.1990
(51) Int. Cl.: C07C 229/28

(54) **Verfahren zur Stabilisierung einer festen pharmazeutischen Zubereitungsform von cyclischen Aminosäuren.**
Process for the stabilisation of a solid pharmaceutical composition comprising cyclic amino acids.
Procédé pour la stabilisation d'une composition pharmaceutique solide contenant des aminoacides cycliques.

(30) Priorität: 25.08.1989 DE 3928183
(43) Veröffentlichungstag der Anmeldung: 27.02.1991
(73) Patentinhaber: GÖDECKE AKTIENGESELLSCHAFT, D-10587 Berlin (DE)
(72) Erfinder: Augart, Helmut, D-7808 Waldkirch (DE); Gebhardt, Uwe, Dr., D-7808 Waldkirch (DE); Herrmann, Wolfgang, Dr., D-7800 Freiburg (DE)

(56) Entgegenhaltungen:
- DE-A- 2 460 891
- DE-A- 2 543 821
- DE-A- 2 557 220
- Advanced Organic Chemistry, Jerry March, second edition, 1977, Seite 353.

## Beschreibung

Aus der DE-OS 24 60 891 sind cyclische Aminosäurederivate der allgemeinen Formel I 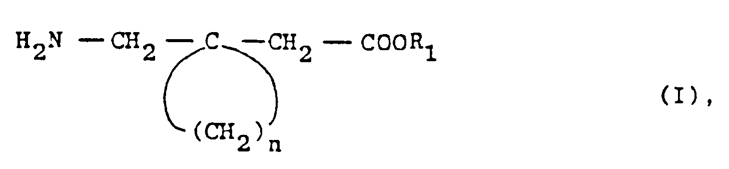
in welcher R₁ Wasserstoff oder eine niedere Alkylgruppe und n die Zahlen 4 bis 6 bedeuten,
sowie deren pharmakologisch verträgliche Salze bekannt. Sie weisen wertvolle pharmakodynamische Eigenschaften auf.

Die Verbindungen der allgemeinen Formel I zeichnen sich durch eine außerordentlich geringe Toxizität aus. Im Tierversuch wurde ein bemerkenswerter Schutzeffekt gegen den durch Thiosemicarbazid induzierten Krampf und gegen den Cardiazolkrampf gefunden. Die Verbindungen sind zur Therapie bestimmter zerebraler Erkrankungen geeignet. So eignen sie sich zur Behandlung bestimmter Formen der Epilepsie, von Schwindelanfällen, der Hypokinese und von Schädeltraumen, und bewirken eine Verbesserung der zerebralen Funktionen. Sie stellen deshalb auch besonders wirksame Geriatrika dar.

Die Verbindungen der allgemeinen Formel I können hergestellt werden, indem man in an sich bekannter Weise entweder
a) eine Verbindung der allgemeinen Formel II 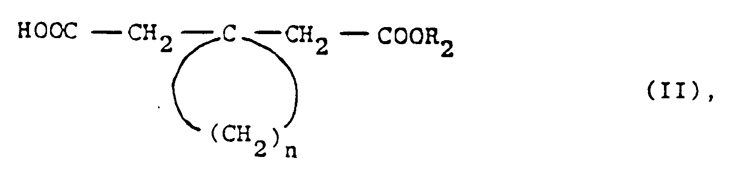 in welcher R₂ eine Alkylgruppe mit bis zu 8 Kohlenstoffatomen darstellt und n die oben genannte Bedeutung hat,
   über ein reaktives Säurederivat in ein Azid überführt und dieses dem sogenannten "Curtius'schen Abbau" unterwirft, oder
b) eine Verbindung der allgemeinen Formel III 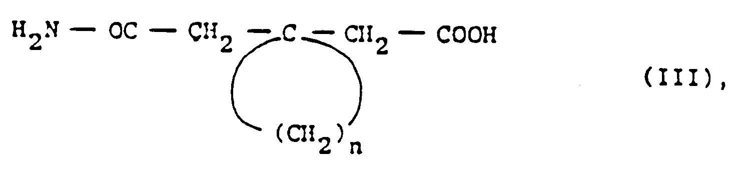 in welcher n die oben genannte Bedeutung hat,
   dem sogenannten "Hofmann'schen Abbau" unterwirft, oder
c) eine Verbindung der allgemeinen Formel IV 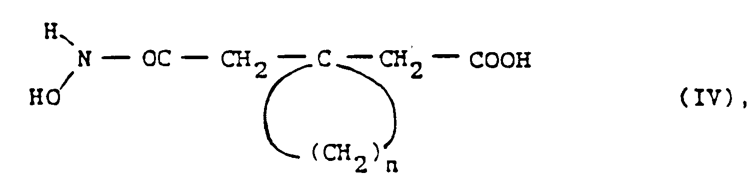 in welcher n die oben genannte Bedeutung hat,
   dem sogenannten "Lossen'schen Abbau" unterwirft, und die so erhaltenen freien Aminosäuren anschließend gewünschtenfalls durch Veresterung in deren niedere Alkylester bzw. durch Umsetzung mit Säuren oder Basen in pharmakologisch verträgliche Salze überführt.

Als besonders potenter Wirkstoff hat sich die Aminomethyl-1-cyclohexanessigsäure (Gabapentin) der Formel Va 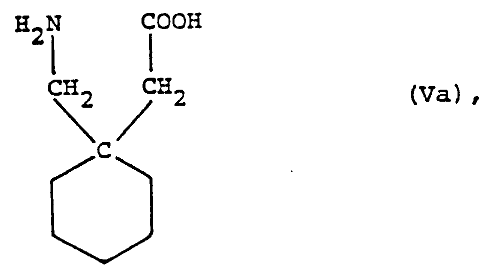
erwiesen. Gabapentin weist strukturell eine gewisse Verwandtschaft zur γ-Aminobuttersäure (GABA) auf, die jedoch nicht in der Lage ist, die Blut-Hirnschranke zu überwinden. Gabapentin besitzt diesen Nachteil nicht und stellt somit ein hochwirksames Anticonvulsivum mit außerordentlich geringer Toxizität dar (Drugs of the Future, 11/6 (1986) S. 518-519).

Andererseits haben sich bei der Herstellung und Lagerung der Verbindungen der Formel (I), in denen R₁ Wasserstoff ist, bisher nur teilweise beherrschbare Probleme gezeigt, die der Entwicklung brauchbarer Darreichungsformen im Wege standen. Zunächst wurde bei der Anwendung der verschiedenen möglichen Herstellungsverfahren festgestellt, daß die erhaltenen Verbindungen ohne ersichtlichen Grund erhebliche Abweichungen im Reinheitsgrad aufwiesen. Durch spezielle, zusätzliche Reinigungsschritte konnte dieses Problem scheinbar zunächst gemeistert werden. Dann zeigte sich jedoch bei Untersuchungen der Langzeitstabilität, daß auch sehr reine Verbindungen (I) mit fortschreitender Lagerung sehr unterschiedliche Stabilitäten aufwiesen. Wiederum war es nicht möglich, eine behebbare Ursache für die mangelhafte Stabilität zu ermitteln, da diese offensichtlich von zunächst unbekannten Bedingungen abhing. Erst eine lange Reihe systematischer Untersuchungen führte zu einer Lösung des Problems, stabile Wirkstoffe und Zubereitungsformen der Verbindungen (I) bereitzustellen.

In der Annahme, das Hydrochlorid von Gabapentin sei die geeignetste Wirkstofform, weil Salze und insbesondere Hydrochloride in aller Regel eine besonders gute Stabilität und eine gute Löslichkeit erwarten lassen, ging man zunächst vom Gabapentin·Hydrochlorid aus. Aber es mußte festgestellt werden, daß dieses in pharmazeutischen Zubereitungen zum Teil noch instabiler war, als die freie Aminosäure.

Nach der Reaktionsfolge 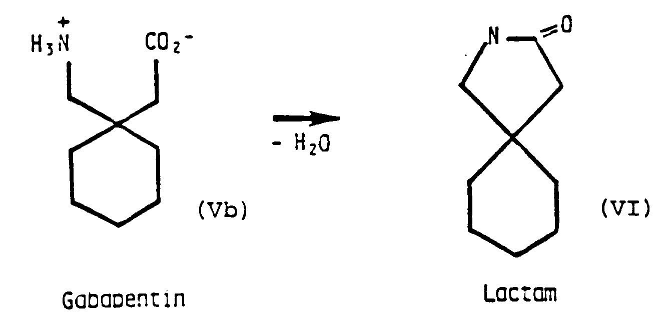
bildet Gabapentin in analoger Weise wie die übrigen Verbindungen der Formel VII 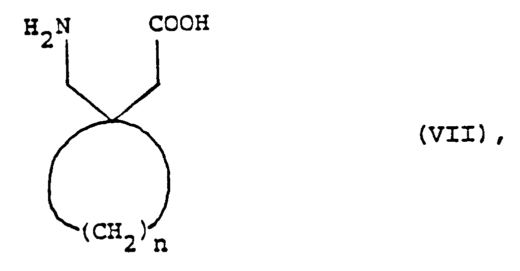
in welcher n die Zahlen 4 bis 6, bevorzugt 5 bedeuten,
das Lactam VI.

Lactame der allgemeinen Formel VIII 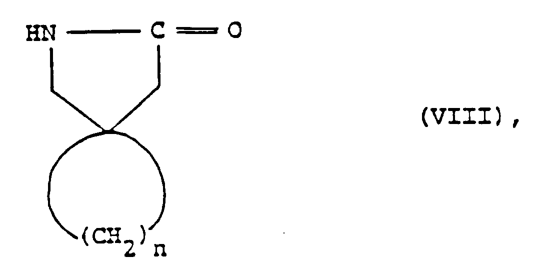
in welcher n die obengenannte Bedeutung hat,
bilden sich jedoch nicht nur bei der Herstellung, sondern überraschend auch bei der Lagerung.
Der Ablauf dieser in fester Phase und unter trockenen Lagerungsbedingungen unerwarteten Reaktion führte durch das bei der Cyclisierung freiwerdende Wasser zusätzlich zu Problemen für die Stabilität trockener Arzneiformen, wie z.B. Tabletten und Kapseln, die unter Feuchtigkeit zum Verkleben bzw. zum Erweichen neigen.

Versuche, den Lactamgehalt des eingesetzten Wirkstoffs von vornherein möglichst niedrig zu halten, führten bei der Herstellung wie bei der Lagerung der Wirksubstanz sowohl in reiner Form als auch in fertigen Zubereitungen zu weiteren zunächst nicht lösbaren Problemen, weil sich herausstellte, daß die erwähnte Cyclisierungsreaktion überraschend auch im alkalischen Bereich auftrat.

Anders als die reinen Wirksubstanzen der Formel (I) weisen die Lactame eine gewisse Toxizität auf, und müssen daher soweit möglich vermieden werden. Während z.B. Gabapentin eine Toxizität (LD₅₀, Maus) von mehr als 8000 mg/kg aufweist, wurde für das entsprechende Lactam VI eine solche von 300 mg/kg ermittelt. Es ist demnach zu fordern, daß diese Verunreinigungen und das potentielle Entstehen solcher Zersetzungsprodukte bei der Lagerung pharmazeutischer Zubereitungen aus Sicherheitsgründen auf ein Minimum reduziert werden.

Bei der Untersuchung fertiger Arzneiformen stellte sich schließlich weiter erschwerend heraus, daß Ursache für die Lactambildung offenbar auch katalytische Effekte von Hilfsstoffen sind, die ebenfalls keiner erkennbaren Logik folgen. Um zu ergründen, welche Hilfsstoffe die Lactambildung fördern, mußten daher langwierige Reihenversuche angestellt werden. Diese ergaben zum Beispiel, daß Poloxamer NF sich völlig neutral verhält und, bei alleiniger Anwesenheit, die Stabilität des Wirkstoffs Gabapentin nicht beeinträchtigt, während bei der Verwendung von Polyethylenglycol (PEG) in erheblichem Umfang Cyclisierung zum Lactam auftrat.
In einer weiteren Versuchsserie mit sehr reinem Wirkstoff zeigte es sich dann jedoch, daß PEG ein durchaus brauchbarer Hilfsstoff ist.

Folgende Hilfsstoffe verminderten beispielsweise die Stabilität der Verbindungen I und sollten bei der Herstellung pharmazeutischer Zubereitungen vermieden werden:
Modifizierte Maisstärke, Croscarmelose-Na, Glycerin-Behensäureester, Methacrylsäurecopolymere (Typ A und C), Anionenaustauscher Titandioxid und Kieselgele, wie z.B. Aerosil 2OO.

Folgende Hilfsstoffe hatten hingegen keinen merklichen Einfluß auf die Stabilität der Verbindungen I:
Hydroxypropyl-methyl-cellulose, Polyvinylpyrrolidon, Crospovidon, Poloxamer 407, Poloxamer 188, Stärkeglykolat-Na, Copolyvidon, Maisstärke, Cyclodextrin, Lactose, Talkum, sowie Copolymer aus Dimethylaminomethacrylsäure und neutralem Methacrylsäureester.

Es sind, um die als zulässig erachtete Obergrenze von 0,5 % (Gew.) Gabapentin-Lactam (bezogen auf Gabapentin) nicht zu überschreiten, und um die Lagerfähigkeit sowohl des Wirkstoffs als auch entsprechender pharmazeutischer Zubereitungsformen sicherzustellen, folgende Maßnahmen einzuhalten:
1.) Die Wirkstoffe der Formel I müssen durch geeignete Maßnahmen als hochreine, nicht derivierte freie Aminosäuren, zum Beispiel aus dem entsprechenden Hydrochlorid durch Ionenaustausch, hergestellt werden. Dabei sollte der Anteil an verbleibenden Hydrochlorid-Beimengungen 20 ppm nicht überschreiten. Entsprechendes gilt auch für andere Mineralsäuren.
2.) Es muß im Falle von pharmazeutischer Zubereitungen durch eine gezielte Auswahl von Hilfsstoffen jede Katalyse der Lactambildung unterbunden werden.
3.) Es muß durch Kontrollen sichergestellt werden, daß die obigen Bedingungen erfüllt sind. Dies ist in der Regel der Fall, wenn die Lactambildung unter den allgemein für Arzneimittel geltenden Lagerbedingungen innerhalb eines Zeitraums von 1 Jahr nach der Herstellung der pharmazeutischen Zubereitungen oder der Wirkstoffe um nicht mehr als 0.2 Gew.%, vorzugsweise 0,1 Gew.% (bezogen auf den reinen Wirkstoff), zunimmt.

Die Erfindung betrifft daher ein Verfahren zur Stabilisierung einer festen pharmazeutischen Zubereitungsform von cyclischen Aminosäuren der allgemeinen Formel VII 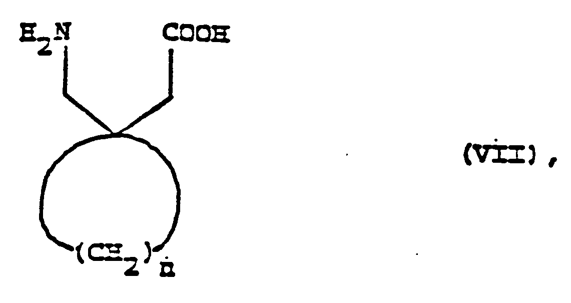
in welcher n die Zahlen 4 bis 6 bedeutet
dadurch gekennzeichnet, daß man eine nicht derivierte Aminosäure VII, die höchstens 0,3 Gew.% Laktam der allgemeinen Formei VIII 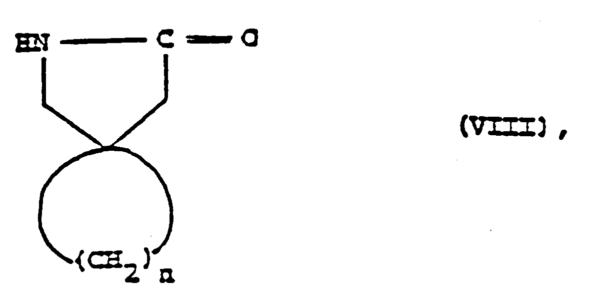
in welcher n die Zahlen 4 bis 6 bedeutet,
und einen Mineralsäureanteil von nicht mehr als 20 ppm aufweist, unter Zugabe von Hydroxypropyl-methylcellulose, Polyvinylpyrrolidon, Crospovidon, Poloxamer 407, Poloxamer 188, Stärkeglykolat-Na, Lactose, Copolyvidon, Maisstärke, Cyclodextrin, Talkum, Copolymer aus Dimethylaminomethacrylsäure und/oder neutralern Methacrylsäureester in eine feste pharmazeutische Zubereitung überführt.

Die Erfindung betrifft weiterhin das obengenannte Verfahren,
das dadurch gekennzeichnet ist, daß
die Verbindung der allgemeinen Formel VIII vor der Überführung in eine pharmazeutische Zubereitung allein oder zusammen mit einer Verbindung der allgemeinen Formel VII mit halbkonzentrierter Mineralsäure bis zur praktisch quantitativen Öffnung des Laktamrings der Formel VIII hydrolysiert und das so erhaltene Reaktionsprodukt durch Ionenaustauschchromatografie von den Anionen der eingesetzten Mineralsäure bis auf einen Anteil von nicht mehr als 20 ppm befreit wird.

Schließlich betrifft die Erfindung pharmazeutische Zubereitungen, die nach dem obigen Verfahren hergestellt worden sind.

### Beispiele

### Beispiel 1

### 1-(Aminomethyl)cyclohexan-essigsäure·Hydrochlorid

22,3 Liter Wasser und 22,3 Liter konzentrierter Salzsäure werden in einem T100-Reaktor gemischt und 6,41 kg Gabapentin-Lactam unter Rühren zugegeben. Die gebildete klare braune Lösung wird anschließend bei 108°C 6 Stunden unter Rückfluß gekocht. Das Reaktionsgemisch wird sich selbst überlassen bis es auf 28°C gekühlt ist. Das dabei ausgefallene weiße Präzipitat wird durch Zugabe weiterer 40 Liter Wasser wieder gelöst. Zur Entfernung noch ungelösten Lactams wird das Reaktionsgemisch dreimal mit je 30 Liter Dichlormethan extrahiert. Die schwach gelbe wässrige Phase wird in einem Vakuum-Evaporator (QVF 100L) zur Trockene eingedampft. Zum Schluß beträgt die Temperatur bei 133 Pa 80°C. Die nahezu trockene Kristallmassee wird mit 12,8 Liter Aceton aufgerührt und abgenutscht.Dann wird sie mit 2 Liter Aceton gewaschen und 4 Stunden bei 60°C getrocknet. Die Ausbeute beträgt ca. 60%.

### Beispiel 2

### 1-(Aminomethyl)cyclohexan-essigsäure

Eine 3 m lange und 200 mm weite Chromatografiesäule wird mit 50 Liter Ionenaustauscherharz (IRA 68) gefüllt. Das Harz wird mit einer Lösung von (14 Liter) konzentriertem wässrigen Ammoniak in 300 Liter demineralisiertem Wasser regeneriert und anschließend mit 150 Liter demineralisiertem Wasser gewaschen. Sobald das Eluat pH 6,8 erreicht und kein Chlorid mehr nachweisbar ist, wird eine Lösung von 8,67 kg (40,8 Mol) 1-Aminomethyl-1-cyclohexan-essigsäure·hydrochlorid in 43 Liter demineralisiertem Wasser in die Säule gegeben. Die freie Aminosäure wird mit demineralisiertem Wasser (1,5 Liter/Minute) eluiert und in 15 Fraktionen von 15 Litern aufgefangen. Die kombinierten Fraktionen werden bei 6,65 KPa und bei höchstens 45°C eingedampft. Der weiße feste Rückstand wird in 20 Liter Methanol gegeben, am Rückfluß erhitzt, filtriert und auf -10°C gekühlt. Das dabei auskristallisierte Produkt wird zentrifugiert, mit 10 Liter kaltem Methanol gewaschen und 17 Stunden bei 30 - 40°C getrocknet.
Man erhält 4,9 kg (71 % d.Th.) reine 1-(Aminomethyl)cyclohexan-essigsäure vom Fp. 165°C. Weitere 0,8 kg lassen sich durch Aufarbeitung der Mutterlaugen gewinnen.

## Patentansprüche

1. Verfahren zur Stabilisierung einer festen pharmazeutischen Zubereitungsform von cyclischen Aminosäuren der allgemeinen Formel VII 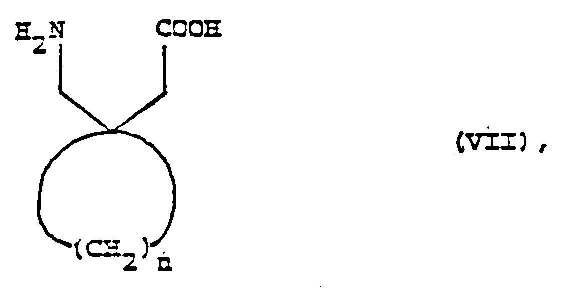 in welcher n die Zahlen 4 bis 6 bedeutet
dadurch gekennzeichnet, daß man eine nicht derivierte Aminosäure VII, die höchstens 0,3 Gew.% Laktam der allgemeinen Formel VIII 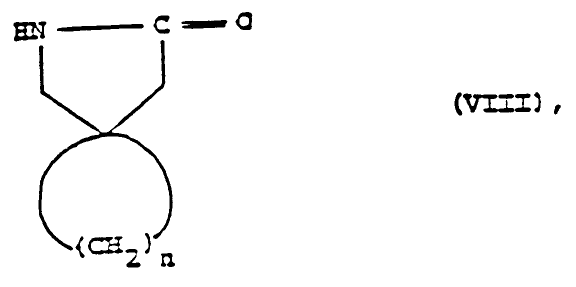 in welcher n die Zahlen 4 bis 6 bedeutet,
und einen Mineralsäureanteil von nicht mehr als 20 ppm aufweist, unter Zugabe von Hydroxypropyl-methylcellulose, Polyvinylpyrrolidon, Crospovidon, Poloxamer 407, Poloxamer 188, Stärkeglykolat-Na, Lactose, Copolyvidon, Maisstärke, Cyclodextrin, Talkum, Copolymer aus Dimethylaminomethacrylsäure und/oder neutralem Methacrylsäureester in eine feste pharmazeutische Zubereitung überführt.

2. Verfahren nach Anspruch 1 , dadurch gekennzeichnet, daß die Mineralsäure Salzsäure ist.

3. Verfahren nach Anspruch 1 oder 2, dadurch gekennzeichnet, daß die Verbindung der allgemeinen Formel VIII vor der Überführung in eine pharmazeutische Zubereitung allein oder zusammen mit einer Verbindung der allgemeinen Formel VII mit halbkonzentrierter Mineralsäure bis zur praktisch quantitativen Öffnung des Laktamrings der Formel VIII hydrolysiert und das so erhaltene Reaktionsprodukt durch Ionenaustauschchromatografie von den Anionen der eingesetzten Mineralsäure bis auf einen Anteil von nicht mehr als 20 ppm befreit wird.

4. Pharmazeutische Zubereitung, hergestellt nach einem der Ansprüche 1 bis 3.

## Claims

1. Process for stabilising a pharmaceutical preparation, in solid form, of cyclic amino acids of the general formula VII 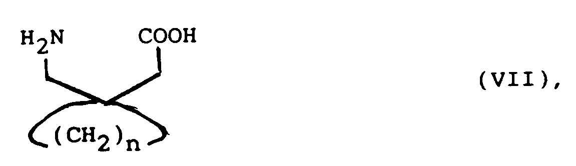 in which n represents the numbers 4 to 6, characterised in that an underived amino acid VII, which has at the most 0.3% by weight lactam of the general formula VIII 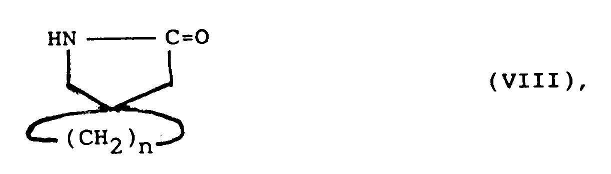 in which n represents the numbers 4 to 6, and a portion of mineral acid of no more than 20 ppm, converts to a solid pharmaceutical preparation with an addition of hydroxypropylmethylcellulose, polyvinylpyrrolidone, crospovidone, poloxamer 407, poloxamer 188, sodium starch glycolate, lactose, copolyvidone, maize starch, cyclodextrin, talcum, co-polymers of dimethylaminomethacrylic acid, and/or neutral methacrylic acid esters.

2. Process according to claim 1, characterised in that the mineral acid is hydrochloric acid.

3. Process according to claim 1 or 2, characterised in that the compound of the general formula VIII, before conversion into a pharmaceutical preparation, alone or with a compound of the general formula VII, is hydrolysed with a semi-concentrated mineral acid up to the practically quantitative opening of the lactam ring of the formula VIII and the reaction product thus obtained is freed from the anions of the mineral acid used by ion exchange chromatography down to a portion of no more than 20 ppm.

4. Pharmaceutical preparation, produced according to one of claims 1 to 3.

## Revendications

1. Procédé de stabilisation d'une forme de préparation pharmaceutique solide d'amino acide cyclique de formule générale VII 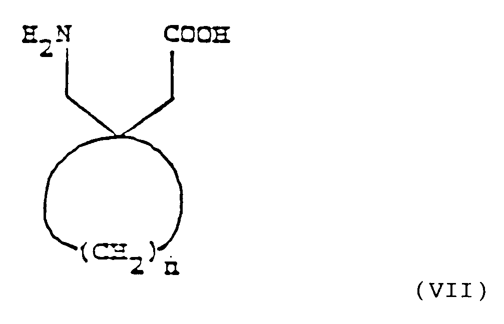 dans laquelle n signifie les nombres de 4 à 6
caractérisé en ce que l'on transforme en une préparation pharmaceutique solide un amino acide VII non dérivé qui contient au maximum 0,3% en poids de lactame de formule générale VIII 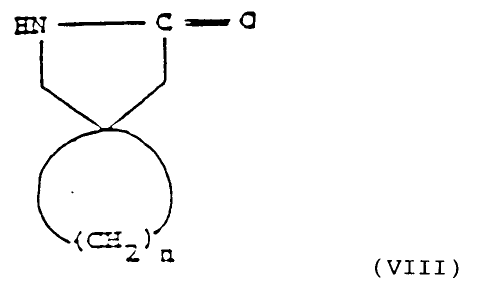 dans laquelle n signifie les nombres 4 à 6, et une proportion d'acide minéral qui ne dépasse pas 20 ppm, par addition d'hydoxypropyl-méthylcellulose, polyvinylpyrrolidon, Crospovidon, Poloxamer 407, Poloxamer 188, Amidon glycolate-Na, lactose, copolyvidon, amidon de maïs, cyclodextrine, talc, copolymère d'acide diméthylaminométhacrylique et/ou d'ester neutre d'acide méthacrylique.

2. Procédé de fabrication selon la revendication 1 caractérisé en ce que l'acide minéral est l'acide chlorydrique.

3. Procédé selon la revendication 1 ou 2 caractérisé en ce que le dérivé de formule générale VIII, avant la transformation en une préparation pharmaceutique, seul ou en même temps qu'un dérivé de formule générale VII, est hydrolysé par un acide minéral semi-concentré jusqu'à ouverture pratiquement quantitative du cycle de lactame de formule VIII et le produit de réaction ainsi obtenu est libéré par chromatographie échangeuse d'ions des anions de l'acide minéral employé jusqu'à concurrence d'une proportion qui ne dépasse pas 20 ppm.

4. Préparation pharmaceutique préparée selon l'une quelconque des revendications 1 à 3.
